(19) Europäisches Patentamt
European Patent Office
Office européen des brevets

(11) Veröffentlichungsnummer: **0 062 821**
**B1**

(12) **EUROPÄISCHE PATENTSCHRIFT**

(45) Veröffentlichungstag der Patentschrift: **22.05.85**

(51) Int. Cl.[4]: **C 07 D 231/18**, A 01 N 47/18

(21) Anmeldenummer: **82102612.7**

(22) Anmeldetag: **29.03.82**

(54) **N-Alkyl-O-pyrazol-4-yl-carbaminsäureester, Verfahren zu ihrer Herstellung und ihre Verwendung als Schädlingsbekämpfungsmittel.**

(30) Priorität: **11.04.81 DE 3114833**

(43) Veröffentlichungstag der Anmeldung: **20.10.82 Patentblatt 82/42**

(45) Bekanntmachung des Hinweises auf die Patenterteilung: **22.05.85 Patentblatt 85/21**

(84) Benannte Vertragsstaaten: **AT BE CH DE FR GB IT LI NL**

(56) Entgegenhaltungen:
**EP - A - 0 023 326**
**CH - A - 282 655**
**DE - A - 2 420 360**
**DE - B - 1 108 202**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**
Erfinder: **Hammann, Ingeborg, Dr., Belfortstrasse 9, D-5000 Koeln 1 (DE)**
Erfinder: **Homeyer, Bernhard, Dr., Obere Strasse 28, D-5090 Leverkusen 3 (DE)**

**EP 0 062 821 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue N-Alkyl-O-pyrazol-4--yl-carbaminsäureester, mehrere Verfahren zu ihrer Herstellung und ihre Verwendung als Schädlingsbekämpfungsmittel, insbesondere als Insektizide und Nematizide.

Es ist bekannt, dass bestimmte Carbaminsäureester, wie z.B. N-Methyl-O-(1-methyl-pyrazol-4-yl)--carbaminsäureester, N-Methyl-O-(1-isopropyl-pyrazol-4-yl)-carbaminsäureester, N-Methyl-O-(1-n--propyl-pyrazol-4-yl)-carbaminsäureester und N--Methyl-O-(1-cyclopentyl-pyrazol-4-yl)-carbaminsäureester pestizid wirksam sind (vergleiche DE-A-29 31 033, welche EP-A-23 326 entspricht).

Die Wirkung dieser Verbindungen ist jedoch, insbesondere bei niedrigen Wirkstoffkonzentrationen und Aufwandmengen, nicht immer ganz zufriedenstellend.

Es wurden nun neue N-Alkyl-O-pyrazol-4-yl-carbaminsäureester der Formel (I) gefunden

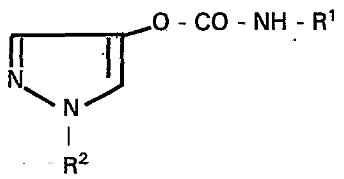

(I)

in welcher

$R^1$ für $C_2$-$C_4$-Alkyl, $C_3$-$C_5$-Alkenyl, $C_3$-$C_5$-Alkinyl oder $C_3$-$C_6$-Cycloalkyl steht,

und

$R^2$ für $C_2$-$C_6$-Alkyl steht.

Man erhält die neuen N-Alkyl-O-pyrazol-4-yl-carbaminsäureester der Formel (I), wenn man 4-Hydroxypyrazole der Formel

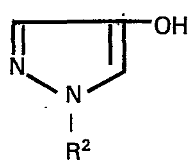

(II)

in welcher

$R^2$ die oben angegebene Bedeutung hat,

a) mit Isocyanaten der Formel (III)

$R^1 - N = C = O$ (III)

in welcher

$R^1$ die oben angegebene Bedeutung hat,

umsetzt, oder

b) gegebenenfalls in Gegenwart eines Verdünnungsmittels und gegebenenfalls in Gegenwart von Säurebindern mit Carbamoylhalogeniden der Formel

$R^1 - NH - CO - Hal$ (IV)

in welcher

$R^1$ die oben angegebene Bedeutung hat und

Hal für Fluor oder Chlor steht,

umsetzt.

Die neuen N-Alkyl-O-pyrazol-4-yl-carbaminsäureester der Formel (I) zeichnen sich durch hohe Wirksamkeit als Schädlingsbekämpfungsmittel, insbesondere durch hohe insektizide und nematizide Wirksamkeit aus.

Überraschenderweise zeigen die erfindungsgemässen Verbindungen der Formel (I) erheblich höhere insektizide und nematizide Wirkung als bekannte Verbindungen ähnlicher Konstitution und gleicher Wirkungsrichtung. Besonders hervorzuheben ist ihre Wirkung gegen Insekten aus der Ordnung der Lepidoptera wie z.B. Heliothis spp.

Die erfindungsgemässen N-Alkyl-O-pyrazol-4-yl--carbaminsäureester sind durch die Formel (I) allgemein definiert. In dieser Formel steht $R^1$ vorzugsweise für $C_2$-$C_4$-Alkyl, Propargyl, Allyl oder Cyclopropyl und $R^2$ für $C_2$-$C_6$-Alkyl. Ganz besonders bevorzugt sind diejenigen N-Alkyl-O-pyrazol-4-yl-carbaminsäureester der Formel (I) in denen $R^1$ für Ethyl, iso-Propyl, n-Propyl, n-Butyl, iso-Butyl, tert.-Butyl, Allyl, Propargyl, Cyclopropyl steht; $R^2$ für Ethyl, iso--Propyl, n-Propyl, n-Butyl, iso-Butyl, tert.-Butyl, 1,1-Dimethylpropyl steht.

Ferner sind besonders bevorzugt Verbindungen der Formel (I), in welcher

$R^1$ für Ethyl steht und

$R^2$ die als besonders bevorzugt angegebenen Bedeutungen hat.

Ferner sind besonders bevorzugt Verbindungen der Formel (I), in welcher

$R^1$ für Propyl steht und

$R^2$ die als besonders bevorzugt angegebenen Bedeutungen hat.

Ferner sind besonders bevorzugt Verbindungen der Formel (I), in welcher

$R^2$ für Propyl steht und

$R^1$ die als besonders bevorzugt angegebenen Bedeutungen hat.

Ferner sind besonders, bevorzugt Verbindungen der Formel (I), in welcher

$R^2$ für Butyl steht und

$R^1$ die als besonders bevorzugt angegebenen Bedeutungen hat.

Bevorzugte Einzelverbindungen sind die bei den Herstellungsbeispielen genannten Verbindungen.

Verwendet man als Ausgangsstoffe beispielsweise 1-Iso-propyl-4-hydroxypyrazol und n-Propylisocyanat, so kann der Reaktionsablauf durch das folgende Formelschema wiedergegeben werden (Verfahrensvariante a):

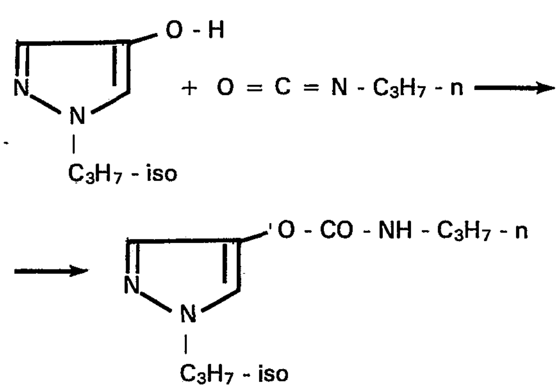


Verwendet man 1-tert.-Butyl-4-hydroxy-pyrazol und N-Ethylcarbamoylfluorid als Ausgangsstoffe, so kann der Reaktionsablauf durch das folgende Formelschema wiedergegeben werden (Verfahrensvariante b):

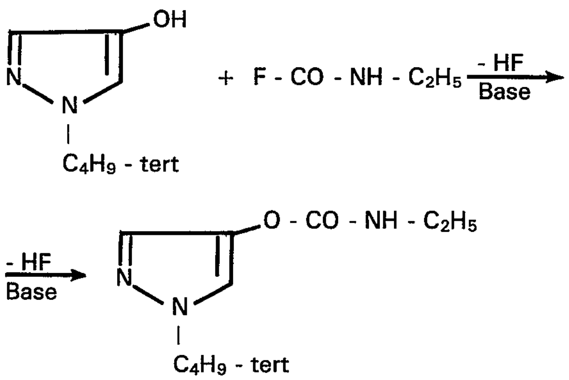


Die als Ausgangsstoffe zu verwendenden 4-Hydroxy-pyrazole sind durch die Formel (II) allgemein definiert. In dieser Formel steht $\underline{R}^2$ vorzugsweise für diejenigen Reste, die bereits in Zusammenhang mit der Beschreibung der erfindungsgemässen Stoffe der Formel (I) vorzugsweise für diese Substituenten genannt wurden.

Als Beispiele seien genannt:

1-Ethyl-, 1-n-Propyl-, 1-iso-Propyl-, 1-n-Butyl-, 1-iso-Butyl-, 1-tert.-Butyl-, 1-(1,1-Dimethylpropyl)-, 4-Hydroxy-pyrazol.

Die 4-Hydroxy-pyrazole der Formel (II) sind teilweise bekannt (vergleiche Liebigs Ann. Chemie <u>313</u>, (1900), 17). Man erhält sie beispielsweise durch Umsetzung von bekannten 4-Methoxy-pyrazolen mit Bromwasserstoffsäure. Die Herstellung der 4--Methoxy-pyrazole erfolgt auf bekannte Weise aus Hydrazin und 2-Methoxy-4-dimethylamino-acrolein (vergleiche Archiv der Pharmazie <u>300</u> (1967), 704-708).

Die weiterhin für das erfindungsgemässe Verfahren (a) als Ausgangsstoffe zu verwendenden Isocyanate sind durch die Formel (III) allgemein definiert. In dieser Formel steht $\underline{R}^1$ vorzugsweise für diejenigen Reste, die bereits im Zusammenhang mit der Beschreibung der erfindungsgemässen Stoffe der Formel (I) vorzugsweise für diesen Substituenten genannt wurden.

Als Beispiele seien genannt:

N-Ethyl-, N-iso-Propyl-, N-n-Propyl-, N-n-Butyl-, N-iso-Butyl-, N-tert.-Butyl-, N-Allyl-, N-Propargyl-, N-Cyclopropyl-, N-Cyclobutyl-, N-Cyclopentyl- und N-Cyclohexyl-isocyanat.

Die Isocyanate der Formel (III) sind bekannt oder lassen sich nach allgemein üblichen und bekannten Verfahren herstellen.

Die für das erfindungsgemässe Verfahren (b) als Ausgangsstoffe zu verwendenden Carbamoylhalogenide sind durch die Formel (IV) allgemein definiert. In dieser Formel steht $\underline{R}^1$ vorzugsweise für die Reste, die bereits im Zusammenhang mit der Beschreibung der erfindungsgemässen Stoffe der Formel (I) vorzugsweise für diese Substituenten genannt wurden. $\overline{Hal}$ steht vorzugsweise für Fluor oder Chlor.

Als Beispiele seien genannt:

N-Ethyl-, N-iso-Propyl-, N-n-Propyl-, N-n-Butyl-, N-iso-Butyl-, N-tert.-Butyl-, N-Allyl-, N-Propargyl-, N-Cyclopropyl-, N-Cyclobutyl-, N-Cyclopentyl- sowie N-Cyclohexyl-carbamoyl-fluorid bzw. -chlorid.

Die Carbamoylhalogenide der Formel (IV) sind bekannt oder lassen sich nach allgemein üblichen und bekannten Verfahren herstellen.

Als Verdünnungsmittel kommen für die Umsetzung gemäss Verfahrensvariante (a) vorzugsweise alle inerten organischen Lösungsmittel in Frage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethylether und Dibutylether, Tetrahydrofuran und Dioxan, Ketone wie Aceton, N-Methylethyl- und Methylisopropyl- und Methylisobutylketon sowie Nitrile, wie Acetonitril und Propionitril.

Das Herstellungsverfahren (a) wird gegebenenfalls unter Verwendung eines Katalysators durchgeführt. Als Katalysatoren können hierbei insbesondere aliphatische, aromatische, oder heterocyclische Amine, wie z.B. Triethylamin, Trimethylamin, Dimethylanilin, Dimethylbenzylamin, Pyridin, Diazabicyclooctan, Diazabicyclononan und Diazabicycloundecen verwendet werden.

Die Reaktionstemperatur kann innerhalb eines grösseren Bereiches variiert werden. Im allgemeinen arbeitet man zwischen 0 und 100°C, vorzugsweise zwischen 10 und 80°C.

Das Verfahren (a) wird im allgemeinen bei Normaldruck durchgeführt. Zur Durchführung des erfindungsgemässen Verfahrens (a) setzt man auf 1 Mol 4-Hydroxy-pyrazol der Formel (II) gewöhnlich zwischen 1 und 1,5 Mol, vorzugsweise zwischen 1 und 1,2 Mol Isocyanat der Formel (III) ein. Die Umsetzung wird vorzugsweise unter Verwendung eines der oben genannten Katalysatoren in einem der oben angegebenen Verdünnungsmittel durchgeführt. Das Reaktionsgemisch wird mehrere Stunden bei der erforderlichen Temperatur gerührt. Dann wird das Lösungsmittel im Vakuum abdestilliert. Man erhält so die Produkte in öliger oder kristalliner Form. Zur Charakterisierung dient der Schmelzpunkt oder der Brechungsindex.

Für die Durchführung gemäss Verfahrensvariante (b) kommen als Verdünnungsmittel vorzugsweise alle inerten organischen Lösungsmittel in Frage. Hierzu gehören vorzugsweise die bei Verfahren (a) aufgezählten Solventien.

Die Umsetzung nach Verfahren (b) wird in Gegenwart von Säurebindern vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triethylamine, N,N-Dimethyl-benzylamin, weiterhin Pyridin sowie 1,4-Diazabicyclo[2,2,2]-octan und 1,5-Diazabicyclo[4,3,0]-non-5-en.

Die Reaktionstemperaturen des Verfahrens (b) können in einem grösseren Bereich variiert werden. Im allgemeinen arbeitet man zwischen 0 und 100°C, vorzugsweise zwischen 10 und 80°C.

Das Verfahren (b) wird im allgemeinen bei Normaldruck durchgeführt. Zur Durchführung des erfindungsgemässen Verfahrens (b) setzt man auf 1 Mol 4-Hydroxy-pyrazol der Formel (II) gewöhnlich zwischen 1 und 1,5 Mol, vorzugsweise zwischen 1 und 1,2 Mol Carbamoylhalogenid der Formel (IV) ein.

Die Umsetzung wird vorzugsweise unter Verwendung eines der oben genannten Säurebindern in einem der oben angegebenen Verdünnungsmittel durchgeführt. Das Reaktionsgemisch wird mehrere Stunden bei der erforderlichen Temperatur gerührt. Dann wird das Lösungsmittel im Vakuum abdestilliert. Man erhält so die Produkte in öliger oder kristalliner Form. Zur Charakterisierung dient der Schmelzpunkt oder der Brechungsindex.

Die erfindungsgemässen N-Alkyl-O-pyrazol-4-yl--carbaminsäureester zeichnen sich durch hervorragende insektizide und nematizide Wirksamkeit aus.

Die erfindungsgemässen Verbindungen können ausserdem eingesetzt werden bei der Bekämpfung von pilzlichen und bakteriellen Pflanzenkrankheiten.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularits, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attegenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Zu den pflanzenparasitären Nematoden gehören Pratylenchus spp., Radophulus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe

mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalino, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe sowie durch Mikroorganismen hergestellte Stoffe.

Die erfindungsgemässe Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepassten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

*Herstellungsbeispiele*

*Beispiel 1*

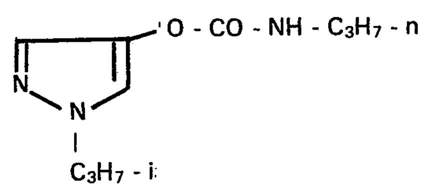

(Verfahrensvariante a)

Zu einer Lösung von 12,6 g (0,1 Mol) 1-Isopropyl-hydroxy-pyrazol und 3 Tropfen Triethylamin in 50 ml Aceton tropft man 8,5 g (0,1 Mol) n-Propylisocyanat. Dann rührt man das Reaktionsgemisch 4 Stunden bei 40°C nach und destilliert das Lösungsmittel im Vakuum ab. Man erhält 20 g (95% der Theorie) N-n-Propyl-O-(1-isopropyl-pyrazol-4-yl)-carbaminsäureester als braunes Öl mit einem Brechungsindex von $n_D^{22} = 1{,}4885$.

*Beispiel 2*

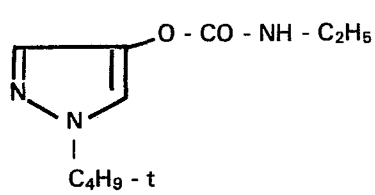

(Verfahrensvariante b)

Eine Mischung aus 7 g (0,05 Mol) 1-tert.-Butyl-4-hydroxypyrazol, 10,4 g (0,075 Mol) Kaliumcarbonat und 100 ml Acetonitril wird mit 4,6 g (0,05 Mol) N-Ethylcarbamoylfluorid versetzt. Das Gemisch wird noch 3 Stunden bei 20°C gerührt. Anschliessend wird abgesaugt. Das Filtrat wird im Vakuum eingedampft. Man erhält 9 g (85% der Theorie) N-Ethyl-O--(1-tert.-butyl-pyrazol-4-yl)-carbaminsäureester in Form beiger Kristalle mit einem Schmelzpunkt von 77°C.

Entsprechend Beispiel 1 und 2 sowie entsprechend den Verfahrensvarianten (a) und (b) werden die Verbindungen der allgemeinen Formel

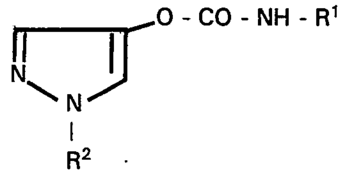

(I)

erhalten:

| Beispiel Nr. | $R^1$ | $R^2$ | Physikalische Konstante |
|---|---|---|---|
| 3 | $C_3H_7$ - iso | $C_3H_7$ - iso | Fp: 54°C |
| 4 | $C_3H_7$ - iso | $C_4H_9$ - tert | Fp: 67°C |
| 5 | $C_2H_5$ | $C_3H_7$ - iso | $n_D^{20}$ : 1,4904 |
| 6 | $C_3H_7$ - n | $C_4H_9$ - sec. | |
| 7 | $C_2H_5$ | - $C(CH_3)_2$ - $CH_2CH_3$ | Fp: 93°C |
| 8 | $C_3H_7$ - n | - $C(CH_3)_2$ - $CH_2CH_3$ | Fp: 71°C |
| 9 | $C_2H_5$ | $C_3H_7$ - n | |
| 10 | $C_3H_7$ - iso | - $C(CH_3)_2$ - $CH_2CH_3$ | |
| 11 | $C_2H_5$ | $C_2H_5$ | $n_D^{23}$ : 1,4919 |
| 12 | $CH_2$=CH - $CH_2$ - | $C_3H_7$ - iso | $n_D^{26}$ : 1,5018 |
| 13 | CH ≡ C - $CH_2$ - | $C_3H_7$ - iso | $n_D^{24}$ : 1,5059 |
| 14 | 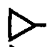 | $C_4H_9$ - tert | Fp: 70°C |
| 15 | 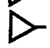 | - $C(CH_3)_2$ - $CH_2CH_3$ | |
| 16 | $CH_2$=CH - $CH_2$ - | $C_4H_9$ - tert | Fp: 53°C |
| 17 | CH ≡ C - $CH_2$ - | $C_4H_9$ - tert | Fp: 93°C |
| 18 | 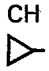 | $C_3H_7$ - iso | Fp: 47°C |
| 19 | $CH_2$=CH - $CH_2$ - | - $C(CH_3)_2$ - $CH_2CH_3$ | |
| 20 | CH ≡ C - $CH_2$ - | - $C(CH_3)_2$ - $CH_2CH_3$ | |
| 21 | $CH_2$=CH - $CH_2$ - | $C_4H_9$ - sec. | |
| 22 | CH ≡ C - $CH_2$ - | $C_4H_9$ - sec. | |
| 23 | 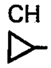 | $C_4H_9$ - sec. | |
| 24 | $CH_2$=CH - $CH_2$ - | $C_2H_5$ | |
| 25 | CH ≡ C - $CH_2$ - | $C_2H_5$ | |
| 26 | 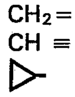 | $C_2H_5$ | |
| 27 | $C_2H_5$ | $C_4H_9$ - sec. | $n_D^{23}$ : 1,4882 |

*Herstellung der Ausgangsprodukte*

*Ausgangsprodukt 1*

Die als Ausgangsmaterialien zu verwendenden 4-Hydroxy-pyrazole können z.B. wie folgt hergestellt werden:

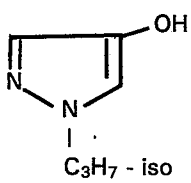

Eine Lösung von 27,6 g (0,2 Mol) 1-iso-Propyl-4--methoxy-pyrazol (Darstellung s. Archiv der Pharmazie <u>300</u> (1967), S. 704-708) in 100 ml 48%iger Bromwasserstoffsäure wird 9 Stunden unter Rückfluss gekocht. Dann destilliert man die überschüssige Säure im Vakuum ab, löst den Rückstand in 40 ml Wasser und neutralisiert die Lösung durch Zugabe von festem Natriumhydrogencarbonat. Anschliessend extrahiert man 6mal mit je 40 ml Chloroform, trocknet die vereinigten Auszüge über Natriumsulfat und destilliert das Lösungsmittel im Vakuum ab. Zurück bleiben 18,6 g (75% der Theorie) 1-iso-Propyl--4-hydroxy-pyrazol in Form beiger Kristalle mit dem Schmelzpunkt 63°C.

In analoger Weise werden die übrigen 4-Hydroxy--pyrazole der Formel (II) hergestellt.

*Ausgangsprodukt 2*

Die teilweise bekannten 4-Methoxypyrazole, die als Vorprodukte für die 4-Hydroxy-pyrazole eingesetzt werden, können z.B. wie folgt hergestellt werden:

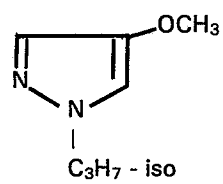

Eine Gemisch von 129 g (1 Mol) 2-Methoxy-3-dimethyl-aminoacrolein (Darstellung s. Archiv der Pharmazie 300 (1967), S 704-708), 123 g (1 Mol) Isopropylhydrazinhemisulfat und 1,1 Mol einer Lösung von Natriummethylat in 400 ml Methanol wird 24 Stunden unter Rückfluss gekocht. Dann destilliert man das Lösungsmittel im Vakuum ab, versetzt den Rückstand mit 500 ml Wasser und extrahiert dann mit 1 l Chloroform. Die organische Lösung wird mit 500 ml Wasser gewaschen, über Natriumsulfat getrocknet und dann bei 30°C im Vakuum eingedampft. Den Rückstand destilliert man im Vakuum und erhält so 82,5 g (60% der Theorie) 1-Isopropyl-4-methoxypyrazol in Form eines gelben Öls mit dem Siedepunkt 94-96°C/14 Torr (1762 Pa).

In analoger Weise werden die übrigen 4-Methoxy-pyrazole hergestellt.

*Verwendungsbeispiele*

In den nachfolgenden Beispielen werden folgende Verbindungen als Vergleichsubstanzen eingesetzt:

(A) 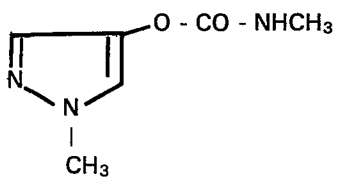

N-Methyl-O-(1-methyl-pyrazol-4-yl)-carbaminsäureester
oder

(B) 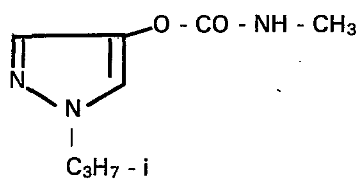

N-Methyl-O-(1-iso-propyl-pyrazol-4-yl)-carbaminsäureester
oder

(C) 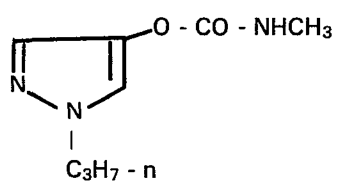

N-Methyl-O-(1-n-propyl-pyrazol-4-yl)-carbaminsäureester
oder

(D) 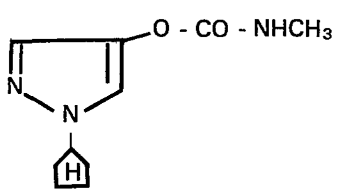

N-Methyl-O-(1-cyclopentyl-pyrazol-4-yl)-carbaminsäureester.

*Beispiel A*

Phaedon-Larven-Test
Lösungsmittel: 3 Gewichtsteile Dimethylformamid
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, dass alle Käfer-Larven abgetötet wurden; 0% bedeutet, dass keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2, 4 und 5.

*Beispiel B*

Heliothis virescens - Test
Lösungsmittel: 3 Gewichtsteile Dimethylformamid
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in % bestimmt. Dabei bedeutet 100%, dass alle Raupen abgetötet wurden; 0% bedeutet, dass keine Raupen abgetötet wurden.

Bei diesem Test zeigen z.B. die folgenden Verbindungen der Herstellungsbeispiele überlegene Wirksamkeit gegenüber dem Stand der Technik: 1, 2 und 5.

*Beispiel C*

Grenzkonzentrations-Test / Bodeninsekten
Phorbia antiqua-Maden
Testinsekt: (im Boden)
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des

Wirkstoffs in der Zubereitung praktisch keine Rolle, entscheidend ist allein die Wirkstoffgewichtsmenge pro Volumeneinheit Boden, welche in ppm ( = mg/l) angegeben wird. Man füllt den Boden in Töpfe und lässt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2, 4 und 5.

*Beispiel D*

Grenzkonzentrations-Test / Wurzelsystemische Wirkung
Testinsekt: Phaedon cochleariae-Larven
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm ( = mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschliesslich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100%, wenn alle Testtiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 4 und 5.

*Beispiel E*

Grenzkonzentrations-Test / Nematoden
Testnematode: Meloidogyne incognita
Lösungsmittel: 3 Gewichtsteile Aceton
Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100%, wenn der Befall vollständig vermieden wird, er ist 0%, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 2 und 4.

**Patentansprüche**

1. N-Alkyl-O-pyrazol-4-yl-carbaminsäureester der Formel (I)

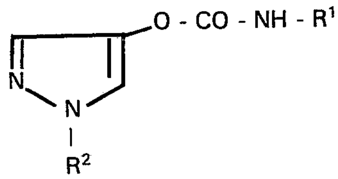

(I)

in welcher
$R^1$ für $C_2$-$C_4$-Alkyl, $C_3$-$C_5$-Alkenyl, $C_3$-$C_5$-Alkinyl oder $C_3$-$C_6$-Cycloalkyl steht,
und
$R^2$ für $C_2$-$C_6$-Alkyl steht.

2. N-Alkyl-O-pyrazol-4-yl-carbaminsäureester der Formel (I) gemäss Anspruch 1, in welcher
$R^1$ = $C_2$-$C_4$-Alkyl, Propargyl, Allyl oder Cyclopropyl,
$R^2$ = $C_2$-$C_6$-Alkyl.

3. Verfahren zur Herstellung von N-Alkyl-O-pyrazol-4-yl-carbaminsäureester der Formel (I) gemäss Anspruch 1, dadurch gekennzeichnet, dass man 4-Hydroxypyrazole der Formel

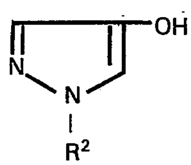

(II)

in welcher
$R^2$ die oben angegebene Bedeutung hat,
a) mit Isocyanaten der Formel (III)

$$R^1 - N = C = O \qquad (III)$$

in welcher
$R^1$ die oben angegebene Bedeutung hat,
umsetzt, oder
b) gegebenenfalls in Gegenwart eines Verdünnungsmittels und gegebenenfalls in Gegenwart von Säurebindern mit Carbamoylhalogeniden der Formel (IV)

$R^1$ - NH - CO - Hal (IV)

in welcher

$R^1$ die oben angegebene Bedeutung hat und

Hal für Fluor oder Chlor steht,

umsetzt.

4. Schädlingsbekämpfungsmittel, gekennzeichnet durch einen Gehalt an mindestens einem N-Alkyl-O-pyrazol-4-yl-carbaminsäureester der Formel (I) gemäss Anspruch 1.

**Claims**

1. N-Alkyl-O-pyrazol-4-yl-carbamic acid esters of the formula (I)

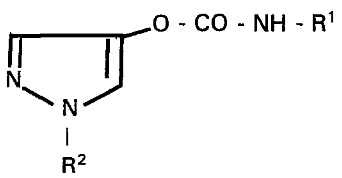

(I)

in which

$R^1$ represents $C_2$-$C_4$-alkyl, $C_3$-$C_5$-alkenyl, $C_3$-$C_5$-alkinyl or $C_3$-$C_6$-cycloalkyl, and

$R^2$ represents $C_2$-$C_6$-alkyl.

2. N-Alkyl-O-pyrazol-4-yl-carbamic acid esters of the formula (I) according to claim 1, in which

$R^1$ = $C_2$-$C_4$-alkyl, propargyl, allyl or cyclopropyl, and

$R^2$ = $C_2$-$C_6$-alkyl.

3. Process for the preparation of N-alkyl-O-pyrazol-4-yl-carbamic acid esters of the formula (I) according to claim 1, characterised in that 4-hydroxypyrazoles of the formula

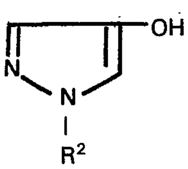

(II)

in which

$R^2$ has the abovementioned meaning,

a) are reacted with isocyanates of the formula (III)

$R^1 - N = C = O$ (III)

in which

$R^1$ has the abovementioned meaning, or

b) are reacted with carbamoyl halides of the formula (IV)

$R^1$ - NH - CO - Hal (IV)

in which

$R^1$ has the abovementioned meaning and

Hal represents fluorine or chlorine,

if appropriate in the presence of a diluent and if appropriate in the presence of acid binders.

4. Agents for combating pests, characterised in that they contain at least one N-alkyl-O-pyrazol-4-yl-carbamic acid ester of the formula (I) according to claim 1.

**Revendications**

1. Esters de O-pyrazole-4-yle d'acides N-alkyl-carbamiques de formule (I)

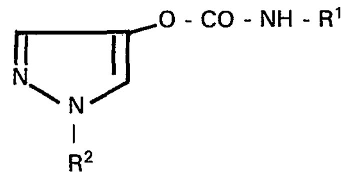

(I)

dans laquelle

$R^1$ est un groupe alkyle en $C_2$ à $C_4$, alcényle en $C_3$ à $C_5$, alcynyle en $C_3$ à $C_5$ ou cycloalkyle en $C_3$ à $C_6$, et

$R^2$ est un groupe alkyle en $C_2$ à $C_6$.

2. Esters de O-pyrazole-4-yle d'acides N-alkyl-carbamiques de formule (I) suivant la revendication 1, dans laquelle

$R^1$ est un groupe alkyle en $C_2$ à $C_4$, propargyle, allyle ou cyclopropyle,

$R^2$ est un groupe alkyle en $C_2$ à $C_6$.

3. Procédé de production d'esters de O-pyrazole-4-yle d'acides N-alkyl-carbamiques de formule (I) suivant la revendication 1, caractérisé en ce qu'on fait réagir des 4-hydroxypyrazoles de formule

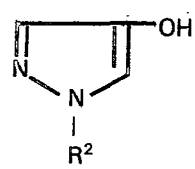

(II)

dans laquelle

$R^2$ a la définition donnée ci-dessus,

a) avec des isocyanates de formule (III)

$R^1 - N = C = O$ (III)

dans laquelle

$R^1$ a la définition indiquée ci-dessus, ou

b) le cas échéant, en présence d'un diluant et en la présence éventuelle d'accepteurs d'acides, avec des halogénures de carbamoyle de formule (IV)

$R^1$ - NH - CO - Hal (IV)

dans laquelle

$R^1$ a la définition indiquée ci-dessus et

Hal représente du fluor ou du chlore.

4. Compositions pesticides, caractérisées par une teneur en au moins un ester de O-pyrazole-4-yle d'acide N-alkyl-carbamique de formule (I) suivant la revendication 1.